(19) Europäisches Patentamt
European Patent Office
Office européen des brevets

(11) Publication number: 0 004 460 B1

(12) # EUROPEAN PATENT SPECIFICATION

(45) Date of publication of patent specification: 09.03.83

(21) Application number: 79300456.5

(22) Date of filing: 22.03.79

(51) Int. Cl.³: C 07 D 241/08, C 07 D 241/44 //C07D243/08, C08K5/34

(54) **Improved synthesis of 1,4-diaza-2-cycloalkanone and substituted derivatives thereof.**

(30) Priority: 24.03.78 US 889751

(43) Date of publication of application: 03.10.79 Bulletin 79/20

(45) Publication of the grant of the patent: 09.03.83 Bulletin 83/10

(84) Designated Contracting States: BE DE FR GB IT NL

(56) References cited:
DE - A - 2 321 526
DE - A - 2 606 819
US - A - 3 920 659
US - A - 3 928 330
US - A - 4 007 157

(73) Proprietor: The B.F. GOODRICH Company
Dept. 0015 WHB-6 500 South Main Street
Akron, Ohio 44318 (US)

(72) Inventor: Lai, John Ta-Yuan
663 Tollis Parkway
Broadview Heights Ohio 44147 (US)

(74) Representative: Shipton, Gordon Owen et al,
W.P. THOMPSON & CO. Coopers Building Church Street
Liverpool L1 3AB (GB)

EP 0 004 460 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Improved synthesis of 1,4-diaza-2-cycloalkanone and substituted derivatives thereof (03.07.79)

This invention relates to the synthesis of 1,4-diaza-2-cycloalkanones and substituted derivatives thereof.

The increasing use of polymers in place of the more traditional types of structural materials (e.g. wood, metals) has necessitated the compounding of such polymers with a variety of stabilizers in order to enhance their ability to withstand prolonged exposure to a variety of degradative forces. Degradation of polymers can be caused by exposure to light, heat and/or air. Such degradation is usually manifest by either a partial or total loss of structural integrity, changes in light transmission properties, changes in color, loss or reduction in flexibility and/or resiliency, or any combination of the above phenomenon. As will be appreciated, the stabilizers which are used in conjunction with such polymeric materials must, in addition to providing protection against such degradative changes, also be compatible with the aesthetic properties of the polymeric article and be effective at low concentrations. The economics of the market place dictate that such stabilizers be relatively inexpensive and capable of preparation from readily available starting materials by simple and straightforward synthesis techniques.

The diazacycloalkanones have been found to be highly effective in the stabilization of polymeric materials against the photodegradative forces of ultraviolet light. The efficacy of such materials in the UV stabilization of polymers is described in copending European Patent Applications Nos. 78/00942.8 and 78/00941.0, both filed on September 20th, 1978 and published as nos. 0001284 and 0001560, respectively. Both the aforementioned applications disclose method for the preparation of the compounds useful in the stabilization of polymer compositions. A third copending European patent application, No. 78/00943.6 which was also filed on September 20th, 1978, published as no. 0001561, discloses a variety of techniques for the convenient synthesis of such highly effective UV stabilizer compounds. The principal advantage of the synthesis described in Application No. 78/00943.6 involves the utilization of readily available starting materials, conventional processing apparatus, the absence of hydrogen cyanide and the attendant hazards associated therewith.

As noted in the section of copending application No. 78/00943.6 entitled "Background of the Invention", cycloalkanes have been previously reported as UV stabilizers in the patent literature; see for example OLS 2,315,042; JAP Pat. 7,453,571 and 7,453,572; and U.S. Patents 3,919,234; 3,920,659 and 3,928,330. This same section also discusses in detail the synthesis of structurally similar compounds and the limitations of such synthesis in the preparation of analogues thereof.

As noted in copending Application No. 78/00943.6 the prior art processes discussed therein do not describe convenient techniques for the preparation of poly-substituted 1,4-diaza-cycloalkanones. The limitations apparent in such processes are most pronounced where one attempts to synthesize symmetrically substituted compounds.

By means of the present invention it is possible to synthesize 1,4-diaza-cycloalkanones or symmetrically substituted 1,4-diaza-cycloalkanones from readily available starting materials and without the attendant risk involved in the use of hydrogen cyanide.

According to the present invention a process for the synthesis of a 1,4-diaza-2-cycloalkanone is provided characterised in that a diamine selected from ethylene 1,2-bis($N^1$-(2-methyl-1,2-propane diamine)1), 2,5-dimethylhexane-2,5-bis($N^1$-2-methyl-1,2-propane diamine)1), *o*-phenylene diamine, N,N'-dimethyl-ethylene diamine, $N^1$-alkyl-2-alkyl-1,2-alkane diamines and diamines of the formula

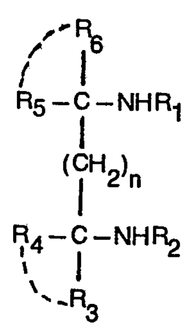

wherein $R_1$ and $R_2$ are independently selected from hydrogen, halogen, alkyl of 1 to 12 carbon atoms, hydroxyalkyl, haloalkyl, cyanoalkyl, aminoalkyl, iminoalkyl, nitrosyl, hydroxyalkoxyalkyl, cyanoalkoxyalkyl, alkenyl and alkoxycarbonyl; $R_3$ to $R_6$ being independently selected from alkyl of 1 to 12 carbon atoms, cycloalkyl, hydroxycycloalkyl, aminoalkyl, iminoalkyl, alkenyl and aralkyl provided further that any two of the above hydrocarbon substituents pendant from the same carbon atom can collectively form a cyclic hydrocarbon and n is 0 to 3 is contacted, in the presence

of a phase transfer catalyst, with a stoichiometric excess of an $\alpha$-trihaloalkylalcohol of the formula

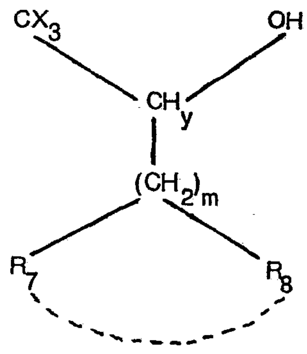

wherein $R_7$ and $R_8$ are selected from the same group of substituents as $R_3$ to $R_6$, X is halogen; m is a number from 0 to 5 and y is 1 except when m is zero in which case y is zero.

In order to control the exotherm of this reaction, it is preferable to chill the reaction vessel. In one of the preferred embodiments of this invention, the reactants and catalyst are contracted with one another while dispersed in a non-polar organic solvent, such as for example dichloromethane.

In a typical embodiment of the process of this invention, an appropriately substituted diamine and an $\alpha$-trichloroalkylalcohol, in the desired relative concentrations, are introduced into a reaction vessel followed thereafter by the addition to said vessel of the phase transformation catalyst dissolved in a non-polar organic solvent and suitably the 1,4-diaza-2-cycloalkanone produced is soluble in the non-polar organic solvent. The reaction vessel is at least partially immersed in an ice bath to control the exotherm of the reaction and additional control can be exercised by the dropwise addition to the reaction medium of predetermined quantities of a highly caustic solution. As a result of the interaction of the materials in the reaction vessel, a water-soluble solid is formed. This solid can be removed from the non-polar reaction medium by filtration or by the addition of water to the reaction vessel followed thereafter by separation of the aqueous phase from the organic fluid layer. The aqueous phase is then further extracted with an organic solvent such as for example chloroform and the combined organic solutions washed with water, dried over sodium sulphate and concentrated. The desired product is crystallized from the combined organic solutions and recrystallized in the conventional fashion to yield a relatively pure product.

Diamines which are especially suitable for use in the process of this invention are the $N^1$-alkyl-2-alkyl-1,2-alkane diamines, e.g. $N^1$-isopropyl-2-methyl-1,2-propane-diamine. Additional 1,2-diamines which are highly effective for use in the process of this invention include $N^1$-propyl-2-methyl-1,2-propanediamine, $N^1$-t-butyl-2-methyl-1,2-propanediamine, $N^1$-t-octyl-2-methyl-1,2-propanediamine, $N^1$-pentyl-2-methyl-1,2-propanediamine, ethylene 1,2-bis-($N^1$-(2-methyl-1,2-propanediamine)1) and 2,5-dimethylhexane-2,5-bis-($N^1$-(2-methyl-1,2-propanediamine)1). In the preferred embodiments of the process of this invention, the $\alpha$-trihaloalkyl alcohol is symmetrically substituted at the $\beta$ carbon. One such material which is especially preferred for use in the process of this invention is 1,1,1-trichloro-2-methyl-2-propanol hydrate. The alcohols which are highly suitable for use in this process include $\alpha$-trichloromethyl-2-propanol, $\alpha$-trichloromethyl-cyclohexanol, and $\alpha$-trichloromethyl-2-butanol.

As noted herein, the above reactants are contacted with one another in the presence of a "phase transfer catalyst". The phrase "phase transfer catalyst" is intended to describe in the context of this invention, any compound, which in the presence of the above reactants, causes a condensation of the alcohol and the diamine in such a fashion so as to result in the formation of a cyclic ketone wherein the oxygen is doubly bonded to an $\alpha$ carbon of the alcoholic reactant.

Materials which have been found to effectively catalyze the condensation of the above reactants in this fashion can be represented by the following formula:

$$Q^+B^-$$

wherein $Q^+$ is $NH_aR_x$, $SH_aR_y$ or $PH_aR_z$ provided that R is alkyl of 1—20 carbon atoms, aryl or aralkyl; a is 0—4, x, y and z are each 0—4 provided further that the sum of a plus x, a plus y, or a plus z equals 4; and $B^-$ is a monovalent anion, such as for example a halide, acetate, perchlorate, sulfate or hydrosulfate.

Catalyst compositions which are preferred for the process of this invention are ammonium salts, for example cetyl trimethylammonium bromide, methyl tricaprylylammonium chloride, cetyl tributyl phosphonium bromide, tetrabutylammonium hydrogen sulfate and tetrabutylammonium chloride.

As noted hereinabove, the catalyst initiation of condensation of the above reactant results in generation of a large exotherm. One means for control of such a large exotherm is to dilute the

reactants in a suitable reaction medium. In the preferred embodiments of this invention, such suitable reaction medium will include inert organic solvents such as dichloromethane, DMF, toluene, THF.

The process of this invention can also be satisfactorily carried out in the absence of solvent; provided, other precautions are taken to control the reaction exotherm.

It is also possible to control the exotherm of the reaction of this process by maintaining the temperature of the reactants at about 10°C or below (e.g. 5 to 10°C). In certain embodiments of this invention, it may also be advisable to add, by dropwise addition, small quantities of caustic aqueous solution (e.g. sodium hydroxide — 50%). The metered addition of caustic serves to neutralize the acidic by-products of the process and thus provides some additional control over the temperature and the rate of product synthesis.

The ratio of reactants to one another in this process is not believed to be critical to the formation of the desired product. However, where one desires to obtain high yields and ease of separation of the reaction product from the reactants and catalysts used in such preparation, it is preferable to adjust the relative concentration of materials so that the molar ratio of diamine to alcohol is in the range of from 0.5 to 1.

The 1,4-diaza-2-cycloalkanones produced in accord with the process of this invention, are highly effective, and in low concentrations, for the stabilization of UV degradable polymeric materials.

Examples

The Examples which follow further define, describe and illustrate the process of this invention. Apparatus and techniques used in this process are standard or as hereinbefore described. Parts and percentages appearing in such Examples are by weight or as otherwise stipulated.

Example 1

$N^1$-isopropyl-2-methyl-1,2-propane diamine (13.0 g, 0.1 mols) and 2-trichloromethyl-2-propanol hydrate (32.1 g, 0.13 mols) were placed in the 3-neck flask which itself was immersed in an ice-bath. 50 milliters dichloromethane and benzyltriethyl ammonium chloride (0.91 g, 0.004 mols) were then added to the flask, followed by the dropwise addition of 50% sodium hydroxide (a total of 48 g or 0.6 mols); the rate of addition being adjusted so as to maintain the reaction temperature below about 10°C. A few hours after the addition of caustic, the reaction was complete, as confirmed by gas-chromatography. Water was added to the flask until all salts were dissolved. The two layers which are formed in the flask, are separated and the aqueous phase extracted once with chloroform. The combined organic solutions were then washed three times with 50 milliters water, dried over sodium sulphate and concentrated. The solid which is formed in the flask was recrystallized from pentane to obtain 9.5 grams of needlelike crystals. The ir spectrum and elemental analysis were consistent with the structure of the desired product.

Example II

The procedures of Example I are repeated except with the following combination of reactants.

| Ex.No. | 1,2-diamine | Alcohol |
|---|---|---|
| II | $N^1$-propyl-2-methyl-1,2-propane diamine | 2-trichloromethyl-2-propanol hydrate |
| III | $N^1$-butyl-4-methyl-2,4-pentane diamine | " |
| IV | N,N′-dimethyl-ethylene diamine | " |
| V | o-phenylene diamine | " |

The foregoing examples have been provided to illustrate some of the preferred embodiments of the process of this invention and not intended to delineate its scope, which is set forth in the following claims.

## Claims

1. A process for the synthesis of a 1,4-diaza-2-cycloalkanone characterised in that a diamine selected from ethylene 1,2-bis ($N^1$-(2-methyl-1,2-propane diamine)1), 2,5-dimethylhexane-2,5-bis($N^1$-2-methyl-1,2-propane diamine)1), *o*-phenylene diamine, N,N′-dimethyl-ethylene diamine, $N^1$-alkyl-2-alkyl-1,2-alkane diamines and diamines of the formula

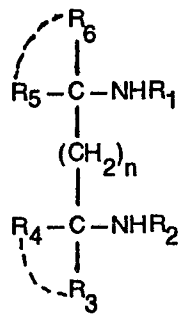

wherein $R_1$ and $R_2$ are independently selected from hydrogen, halogen, alkyl or 1 to 12 carbon atoms, hydroxyalkyl, haloalkyl, cyanoalkyl, aminoalkyl, iminoalkyl, nitrosyl, hydroxyalkoxyalkyl, cyanoalkoxyalkyl, alkenyl and alkoxycarbonyl; $R_3$ to $R_6$ being independently selected from alkyl of 1 to 12 carbon atoms, cycloalkyl, hydroxycycloalkyl, aminoalkyl, iminoalkyl, alkenyl and aralkyl provided further that any two of the above hydrocarbon substituents pendant from the same carbon atom can collectively form a cyclic hydrocarbon and n is 0 to 3 is contacted, in the presence of a phase transfer catalyst, with a stoichiometric excess of an $\alpha$-trihaloalkylalcohol of the formula

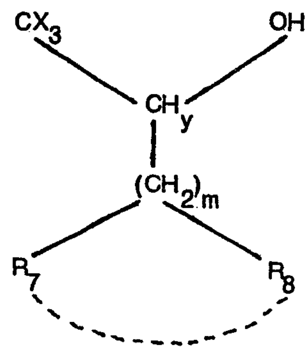

wherein $R^7$ and $R^8$ are selected from the same group of substituents as $R_3$ to $R_6$, X is halogen; m is a number from 0 to 5 and y is 1 except when m is zero in which case y is zero.

2. A process according to claim 1 characterised in that any two of the hydrocarbon substituents pendant from the same carbon atom collectively form an alicyclic hydrocarbon.

3. A process according to claim 1 or 2 characterised in that the temperature of the reactants is maintained 10°C or below.

4. A process according to claim 1, 2 or 3 characterised in that the molar ratio of diamine to alcohol is in the range of from 0.5 to 1.

5. A process according to claim 1, 2, 3 or 4 characterised in that the catalyst has the following formula

$$Q^+ \quad B^-$$

wherein $Q^+$ is $NH_aR_x$, $SH_aR_y$ or $PH_aR_z$ provided that R is alkyl of 1 to 20 carbon atoms, aryl or aralkyl;

a is 0 to 4, x, y and z are each 0 to 4 provided further that the sum of a plus x, a plus y or a plus z equals 4; and

$B^-$ is a monovalent anion.

6. A process according to claim 1 or 2 characterised in that the phase transfer catalyst is an ammonium salt.

7. A process according to claim 5 characterised in that the ammonium salt is cetyl trimethylammonium bromide, methyl tricaprylylammonium chloride, cetyl tributyl phosphonium bromide, tetrabutylammonium hydrogen sulfate, or tetrabutylammonium chloride.

8. A process according to any of claims 1 to 7 characterised in that the reactants and catalyst are contacted in a non-polar organic reaction medium.

9. A process according to claim 8 characterised in that the 1,4-diaza-2-cycloalkanone is soluble in the non-polar organic reaction medium.

10. A process according to any of claims 1 to 9 characterised in that the diamine is a $N^1$-alkyl-2-alkyl-1,2-alkane diamine.

11. A process according to claim 10 characterised in that the diamine is $N^1$-isopropyl-2-methyl-1,2-propanediamine.

12. A process according to any of claims 1 to 11 characterised in that the alcohol is symmetrically substituted at the $\beta$ carbon.

13. A process according to any of claims 1 to 12 characterised in that the alcohol is an $\alpha$-trichloroalkylalcohol.

14. A process according to claim 13 characterised in that the alcohol is 1,1,1-trichloro-2-methyl-2-propanol hydrate.

**Patentansprüche**

1. Verfahren zur Synthese eines 1,4-Diaza-2-cycloalkanons, dadurch gekennzeichnet, daß ein Diamin ausgewählt aus Ethylen-1,2-bis[N[1]-(2-methyl-1,2-propandiamin)1], 2,5-Dimethylhexan-2,5-bis(2-methyl-1,2-propandiamin)-1], o-Phenylendiamin, N,N′-Dimethyl-ethylendiamin, N[1]-Alkyl-2-alkyl-1,2-alkandiaminen und Diaminen der Formel

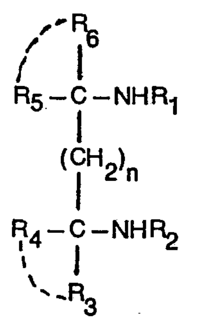

in der
$R_1$ und $R_3$ unabhängig voneinander ausgewählt sind aus Wasserstoff, Halogen, Alkyl mit 1 bis 12 Kohlenstoff-Atomen, Hydroxyalkyl, Halogenoalkyl, Cyanoalkyl, Aminoalkyl, Iminoalkyl, Nitrosyl, Hydroxyalkoxyalkyl, Cyanoalkoxyalkyl, Alkenyl und Alkoxycarbonyl,
$R_3$ bis $R_6$ unabhängig voneinander ausgewählt sind aus Alkyl mit 1 bis 12 Kohlenstoff-Atomen, Cycloalkyl, Hydroxycycloalkyl, Aminoalkyl, Iminoalkyl, Alkenyl und Aralkyl, wobei weiterhin die Möglichkeit gegeben ist, daß jeweils zwei beliebige der vorbezeichneten, an das gleiche Kohlenstoff-Atom gebundenen Kohlenwasserstoff-Substituenten gemeinsam einen cyclischen Kohlenwasserstoff bilden können, und
n 0 bis 3 ist,
in Gegenwart eines Phasentransfer-Katalysators in Berührung gebracht wird mit einem stöchiometrischen Überschuß eines $\alpha$-Trihalogenoalkylalkohols der Formel

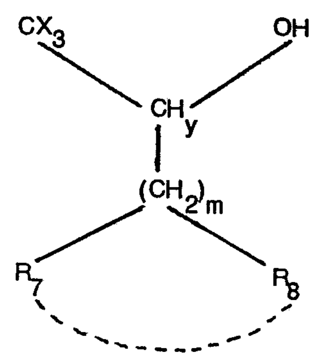

in der
$R_7$ und $R_8$ ausgewählt sind aus der gleichen Gruppe der Substituenten wie $R_3$ bis $R_6$,
X Halogen ist,
m eine Zahl von 0 bis 5 ist und
y 1 ist, ausgenommen in dem Fall, in dem m Null ist, in dem auch y Null ist.

2. Verfahren nach Anspruch 1, dadurch gekennzeichnet, daß jeweils zwei beliebige der an das gleiche Kohlenstoff-Atom gebundenen Kohlenwasserstoff-Substituenten gemeinsam einen alicyclischen Kohlenwasserstoff bilden.

3. Verfahren nach Anspruch 1 oder 2, dadurch gekennzeichnet, daß die Temperatur der Reaktionspartner bei 10°C oder darunter gehalten wird.

4. Verfahren nach Anspruch 1, 2 oder 3, dadurch gekennzeichnet, daß das Stoffmengenverhältnis Diamin zu Alkohol in dem Bereich von 0,5 bis 1 liegt.

5. Verfahren nach Anspruch 1, 2, 3 oder 4, dadurch gekennzeichnet, daß der Katalysator die nachstehende Formel

$Q^+ \quad B^-$

besitzt, in der
$Q^+$ $NH_aR_x$, $SH_aR_y$ oder $PH_aR_z$ ist, wobei R Alkyl mit 1 bis 20 Kohlenstoff-Atomen, Aryl oder Aralkyl ist,
a 0 bis 4 ist,
x, y und z jeweils 0 bis 4 sind,
wobei die Summen (a + x), (a + y) oder (a + z) jeweils gleich 4 sind, und
$B^-$ ein einwertiges Anion ist.

6. Verfahren nach Anspruch 1 oder 2, dadurch gekennzeichnet, daß der Phasentransfer-Katalysator ein Ammonium-salz ist.

7. Verfahren nach Anspruch 5, dadurch gekennzeichnet, daß das Ammoniumsalz Cetyltrimethylammoniumbromid, Methyltricaprylammoniumchlorid, Cetyltributylphosphoniumbromid, Tetrabutylammoniumhydrogensulfat oder Tetrabutyammoniumchlorid ist.

8. Verfahren nach Anspruch 1 bis 7, dadurch gekennzeichnet, daß die Reaktionspartner und der Katalysator in einem nicht-polaren organischen Reaktionsmedium miteinander in Berührung gebracht werden.

9. Verfahren nach Anspruch 8, dadurch gekennzeichnet, daß das 1,4-Diaza-2-cycloalkanon in dem nicht-polaren organischen Reaktionsmedium löslich ist.

10. Verfahren nach Anspruch 1 bis 9, dadurch gekennzeichnet, daß das Diamin ein $N^1$-Alkyl-2-alkyl-1,2-alkandiamin ist.

11. Verfahren nach Anspruch 10, dadurch gekennzeichnet, daß das Diamin $N^1$-Isopropyl-2-methyl-1,2-propandiamin ist.

12. Verfahren nach Anspruch 1 bis 11, dadurch gekennzeichnet, daß der Alkohol an dem $\beta$-Kohlenstoff-Atom symmetrisch substituiert ist.

13. Verfahren nach Anspruch 1 bis 12, dadurch gekennzeichnet, daß der Alkohol ein $\alpha$-Trichloroalkylalkohol ist.

14. Verfahren nach Anspruch 13, dadurch gekennzeichnet, daß der Alkohol 1,1,1-Trichloro-2-methyl-2-propanol-hydrat ist.

## Revendications

1. Procédé pour la synthèse d'une 1,4-diaza-2-cycloalcanone, caractérisé en ce qu'une diamine choisie parmi l'éthylène 1,2-bis ($N^1$(2-méthyl-1,2-propane diamine)1), la 2,5-diméthylhexane-2,5-bis($N^1$-2-méthyl-1,2-propane diamine)1), l'o-phénylène diamine, la N,N'-diméthyl-éthylène diamine, les $N^1$-alcoyl-2-alcoyl-1,2-alcane diamines et les diamines de formule:

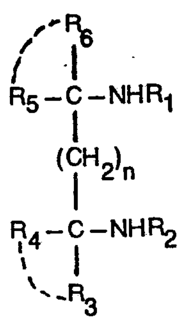

dans laquelle $R_1$ et $R_2$ sont choisis indépendamment parmi l'hydrogène, unhalogène, un groupe alcoyle ayant de 1 à 12 atomes de carbone, hydroxyalcoyle, halogenoalcoyle, cyanoalcoyle, aminoalcoyle, iminoalcoyle, nitrosyle, hydroxyalcoxyalcoyle, cyanoalcoxyalcoyle, alcényle et alcoxycarbonyle; $R_3$ à $R_6$ étant indépendamment choisis parmi des groupes alcoyle ayant de 1 à 12 atomes de carbone, cycloalçoyle, hydroxycycloalcoyle, aminoalcoyle, iminoalcoyle, alcényle et aralcoyle, à condition en outre que deux quelconques des substituants hydrocarbure ci-dessus, fixés sur le même atome de carbone, puissent former ensemble un hydrocarbure cyclique, et n est compris entre 0 et 3, est mise au contact, en présence d'un catalyseur de transfert de phase, avec un excès stoechiométrique d'un $\alpha$-trihalogenoalcoylalcool de formule:

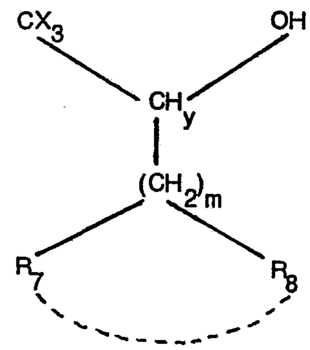

dans laquelle $R_7$ et $R_8$ sont choisis parmi le même groupe de substituants que $R_3$ à $R_6$, X est un halogène; m est un nombre de 0 à 5 et y est l excepté lorsque m est zéro, auquel cas y est zéro.

2. Procédé selon la revendication 1, caractérisé en ce que deux quelconques des substituants hydrocarbure fixés sur le même atome de carbone forment ensemble un hydrocarbure alicyclique.

3. Procédé selon l'une des revendications 1 ou 2, caractérisé en ce que la température des réactifs est maintenue à 10°C ou en-deçà.

4. Procédé selon l'une quelconque des revendications 1 à 3, caractérisé en ce que le rapport molaire de la diamine à l'alcool est de l'ordre de 0,5 à 1.

5. Procédé selon l'une quelconque des revendications 1 à 4, caractérisé en ce que le catalyseur répond à la formule suivante:

$$Q^+ B^-$$

où $Q^+$ est $NH_aR_x$, $SH_aR_y$ $PH_aR_z$, à condition que R soit un groupe alcoyle ayant de 1 à 20 atomes de carbone, aryle ou aralcoyle; a est compris entre 0 et 4, x, y et z sont chacun compris entre 0 et 4, à condition en outre que la somme de a plus x, a plus y ou a plus z soit égale à 4; et $B^-$ est un anion monovalent.

6. Procédé selon l'une des revendications 1 ou 2, caractérisé en ce que le catalyseur de transfert de phase est un sel d'ammonium.

7. Procédé selon la revendication 5, caractérisé en ce que le sel d'ammonium est le bromure de cétyl triméthylammonium, le chlorure de méthyl tricaprylylammonium, le bromure de cétyl tributyl phosphonium, le bisulfate de tétrabutylammonium, ou le chlorure de tétrabutylammonium.

8. Procédé selon l'une quelconque des revendications 1 à 7, caractérisé en ce que les réactifs et le catalyseur sont mis en contact dans un milieu de réaction organique non polaire.

9. Procédé selon la revendication 8, caractérisé en ce que la 1,4-diaza-2-cycloalcanone est soluble dans le milieu de réaction organique non polaire.

10. Procédé selon l'une quelconque des revendications 1 à 9, caractérisé en ce que la diamine est une $N^1$-alcoyl-2-alcoyl-1,2-alcane diamine.

11. Procédé selon la revendication 10, caractérisé en ce que la diamine est la $N^1$-isopropyl-2-méthyl-1,2-propane diamine.

12. Procédé selon l'une quelconque des revendications 1 à 11, caractérisé en ce que l'alcool est symétriquement substitué sur le carbone $\beta$.

13. Procédé selon l'une quelconque des revendications 1 à 12, caractérisé en ce que l'alcool est un $\alpha$-trichloroalcoylalcool.

14. Procédé selon la revendication 13, caractérisé en ce que l'alcool est le 1,1,1-trichloro-2-méthyl-2-pronanol hydraté.